# EUROPÄISCHE PATENTSCHRIFT

(11) **EP 1 405 082 B1**
(45) Veröffentlichungstag und Bekanntmachung des Hinweises auf die Patenterteilung: **18.08.2010**
(21) Anmeldenummer: 02758248.5
(22) Anmeldetag: 19.06.2002
(51) Int. Cl.: G01N 33/68, C07K 14/575, A61K 38/22, C12Q 1/68, C12Q 1/25

(54) **VERFAHREN ZUR IDENTIFIZIERUNG VON VERBINDUNGEN ZUR THERAPIE VON ALTERUNGSPROZESSEN DES HERZ-KREISLAUF SYSTEMS**
METHOD FOR IDENTIFYING COMPOUNDS FOR THE THERAPY OF AGING OF THE CARDIOVASCULAR SYSTEM
PROCEDE D'IDENTIFICATION DE COMPOSES PERMETTANT DE TRAITER LE PROCESSUS DE VIEILLISSEMENT DU SYSTEME CARDIOVASCULAIRE

(30) Priorität: 29.06.2001 DE 10131458
(43) Veröffentlichungstag der Anmeldung: 07.04.2004
(73) Patentinhaber: Sanofi-Aventis Deutschland GmbH, 65929 Frankfurt am Main (DE)
(72) Erfinder: KOSTENIS, Evi, 60594 Frankfurt am Main (DE); BUSCH, Andreas, 65779 Kelkheim (DE); REGITZ-ZAGROSEK, Vera, 10719 Berlin (DE)
(86) Internationale Anmeldenummer: PCT/EP2002/006771
(87) Internationale Veröffentlichungsnummer: WO 2003/003018

(56) Entgegenhaltungen:
- WO-A-01/14888
- DOUGLAS S A ET AL: "Human urotensin-II, the most potent mammalian vasoconstrictor identified to date, as a therapeutic target for the management of cardiovascular disease." TRENDS IN CARDIOVASCULAR MEDICINE. UNITED STATES AUG 2000, Bd. 10, Nr. 6, August 2000 (2000-08), Seiten 229-237, XP002247921 ISSN: 1050-1738
- CHEN MICHELLE M ET AL: "CTGF expression is induced by TGF-beta in cardiac fibroblasts and cardiac myocytes: A potential role in heart fibrosis." JOURNAL OF MOLECULAR AND CELLULAR CARDIOLOGY, Bd. 32, Nr. 10, Oktober 2000 (2000-10), Seiten 1805-1819, XP001153445 ISSN: 0022-2828
- FITZGERALD MELINDA ET AL: "Matrix metalloproteinases can facilitate the heparanase-induced promotion of phenotypic change in vascular smooth muscle cells." ATHEROSCLEROSIS, Bd. 145, Nr. 1, Juli 1999 (1999-07), Seiten 97-106, XP001153446 ISSN: 0021-9150

## Beschreibung

Die Erfindung betrifft ein in vitro Verfahren zur Identifizierung einer Verbindung, welche die Aktivität des Urotensin II Rezeptors modifiziert und dadurch Alterungsprozesse des Herzkreislauf-Systems beeinflußt.

Urotensin II (UII) ist ein auf Blutgefäße einwirkendes Peptid. Es erzeugt je nach Gefäßtyp und betroffener Spezies gefäßerweiternde und gefäßverengende Effekte. Die Aktivität ist in vielerlei Hinsicht mit der von Angiotension II (All) vergleichbar. Urotensin II ebenso wie dessen Rezeptor GPR14 werden im humanen kardiovaskulären System beispielsweise in Endothelzellen, glatten Muskelzellen von Koronararterien, dem Myokard oder dem koronaren Atheroma ausgeprägt. Beiden Faktoren wird deshalb eine wichtige Rolle bei der kardiovaskulären Organisation auf Zellebene und somit in pathophysiologischen Prozessen zugesprochen. Urotensin II ist als natürlicher Ligand von GPR14 bekannt. GPR14 zählt zur Gruppe der GPCRs (G-Protein gekoppelte Rezeptoren).

Versuche an anästhetisierten Affen mit Urotensin II führten zu dramatischen Änderungen der hämodynamischen Parameter. Die Erhöhung des peripheren Widerstands zusammen mit einer Abschwächung der Herzkontraktilität und einem vermindertem Schlagvolumen führte zu einem Zusammenbruch des Herzkreislaufsystems. Vergleichbare Vorgänge laufen auch beim Menschen ab.

G-Protein gekoppelte Rezeptoren (GPCR) spielen eine zentrale Rolle bei einer Vielzahl unterschiedlichster physiologischer Prozesse. Es wird angenommen, daß im menschlichen Genom etwa 1000 Gene für diese Rezeptorfamilie kodieren. Schätzungsweise 40-50 % der gegenwärtig verfügbaren, verschreibungspflichtigen Arzneimittel wirken als Agonisten oder Antagonisten von GPCRs. Dies unterstreicht die bedeutende Rolle dieser Rezeptorklasse für die arzneimittelforschende Industrie. Aufgrund der Größe und Bedeutung der Proteinfamilie und angesichts der Tatsache, daß für viele GPCRs noch kein physiologischer Ligand bekannt sind (orphan GPCRs), ist davon auszugehen, daß diese Rezeptorklasse in Zukunft eines der wichtigsten Reservoirs für geeignete Zielproteine bei der Suche nach neuen Arzneistoffen sein wird.

GPCRs sind eine Familie von integralen Membranproteinen, die auf der Zelloberfläche von Zellen lokalisiert sind. Sie empfangen Signale von extrazellulären Signalstoffen (z.B. Hormone, Neurotransmitter, Peptide, Lipide) und übertragen diese Signale über eine Familie von Guanin-Nukleotid-bindenden Proteinen, sogenannten G-Proteinen, ins Zellinnere. Dabei aktivieren sie in Abhängigkeit von der Spezifität des Rezeptors, des aktivierten G-Proteins und des Zelltyps verschiedene Signaltransduktionswege. Die Polypeptidkette aller GPCRs falten sich zu sieben α-Helices, die die Phospholipid-Doppelschicht der Zellmembran durchspannen. Aufgrund der sieben Membrandurchgänge ergeben sich extra-, und intrazelluläre Loops, die die extrazelluläre Ligandenbindung und die intrazelluläre Kopplung von G-Proteinen ermöglichen. Aus diesem Grund werden GPCRs auch als Sieben-transmembranäre Rezeptoren bezeichnet.

Alle G-Protein gekoppelten Rezeptoren funktionieren nach einem gemeinsamen Grundmuster: die Bindung eines extrazellulären Liganden führt zu einer Konformationsänderung des Rezeptorproteins, so daß dieses Kontakt mit einem G-Protein aufnehmen kann. Über G-Protein-vermittelte Signaltransduktionskaskaden innerhalb der Zelle kommt es letztendlich zu einer biologischen Antwort der Zelle.

G-Proteine sind heterotrimere Proteine, die aus den Untereinheiten α, β und y bestehen und aufgrund von Lipidankern auf der Innenseite der Zellmembran lokalisiert sind. Die Kopplung von aktivierten GPCRs an G-Proteine bewirkt einen GDP/GTP-Austausch der Gα-Untereinheit und die Dissoziation des Heterotrimers in eine α- und eine βγ-Untereinheit. Sowohl die aktivierte α-Untereinheit als auch der βγ-Komplex können intrazelluläre Effektorproteine beeinflussen.

Die Aktivierung der membranständigen Adenylat-Cyclase (AC) durch G-Proteine vom Typ Gαs führt beispielsweise zum Anstieg des intrazellulären cAMP Spiegels bzw. zu dessen Abfall bei Aktivierung von G-Proteinen vom Typ Gαi. G-Proteine vom Typ Gq aktivieren die Phospholipase C (PLC), die die Bildung von Inositol-1,4,5-triphosphat (IP3) und Diacylglycerol (DAG) katalysiert. Diese Moleküle wiederum führen zur Freisetzung von Ca²⁺ aus intrazellulären Speichern, bzw. der Aktivierung der Proteinkinase C (PKC) mit weiteren Effekten in beiden Fällen. Neben den oben genannten G-Protein-Typen (Gαi/s, Gq) existieren noch weitere Typen, die als G16, G12/13 etc. bezeichnet werden. Die Vielfalt dieser G-Proteine spiegelt die Vielzahl unterschiedlichster Funktionen von GPCRs wider.

In der Internationalen Anmeldung WO 01/14888 wird ein Verfahren offenbart, weiches der Identifikation von Agonisten und Antagonisten des Urotenein-II Rezeptors dient. Dieses Verfahren macht sich ein Signal aus der durch den veränderten Rezeptor beeinflussten Signaltransduktionsitaskade zunutze, um den Zustand des Rezeptors bezüglich seiner Interaktion mit einem Agonisten oder Antagonisten zu erfassen.

In der Veröffentlichung von Douglas et al. In TCM Vol. 10, No. 6, 2000 findet sich der Hinweis, dass der humane Urotensin-II Rezeptor die Expression von α₁₋₍₁₎-procollagen mRNA erhöht. Daraus wird gefolgert, dass die Möglichkeit gegeben sein könnte, wie im Falle von anderen Spasmogenen (z.B. ET-1, angiotensin-II, norepinephrine u.a.), dass der Rezeptor in Blutgefässen des Herzens und peripheren Gefäßen Fibrosen erzeugt.

In der Veröffentlichung von Chen et al. in J. Mol. Cell. Cardiol. 32, 1802-1819 (2000) wird dargestellt, dass TGF-β ein spezifischer Regulator von CTGF sowohl in Myozyten und Fibroblasten des Herzens ist. Die Induktion von CTGF durch TFG- β korreliert dabei mit einen signifikanten Anstieg von Fibronectin, Collagen und PAI-1 Infibroblasten des Herzens.

Fitzgerald et al. weist in Atherosclerosis 145, 97-106 (1999) darauf hin, dass heparanase und aktives MMP2 in Kanninchen simultan induziert werden, wenn deren Carotis durch Ballonexperimente gereizt werden und dass in Patienten in Endstadium von komplexen atherosklerotischen Lesionen eine simultane Aktivität der Heparanase, MMP2 und MMP9 beobachtet wird.

Der Urotensin II Rezeptor der Maus ist in WO 00/75168 offenbart. Bekannt sind ebenso Melthoden zur Identifizierung von Agonisten und Antagonisten für GPR14 wie beispielsweise aus der WO 99/40192.
Bislang findet im Stand der Technik hinsichtlich der Screeningmethoden noch keine Beachtung, daß der Urotensin II Rezeptor speziell zum Auffinden solcher Verbindungen verwendet werden kann, die Gene oder Genprodukte, die an Aufbau, Zusammensetzung oder Abbau der extrazellulären Matrix beteiligt sind, einsetzbar ist. Dadurch können gegen Krankheiten, die mit einer Vermehrung von Bindegewebe einhergehen aus pharmazeutischer Sicht nicht effektiv genug entgegen wirkende Substanzen gesucht werden.

Die Erfindung betrifft daher ein Verfahren in vitro zur Identifizierung einer Verbindung, welche die Aktivität des Urotensin II Rezeptors so modifiziert, daß Aktivität oder Menge eines Gens oder Genprodukts, welches an Zusammensetzung, Bildung oder Abbau der extrazellulären Matrix beteiligt ist, beeinflußt werden indem
a] eine Zelle zur Verfügung gestellt wird, in der ein Urotensin II Rezeptor gebildet wird,
b] eine chemische Verbindung zur Verfügung gestellt wird,
c] die Zelle aus a] mit der chemischen Verbindung aus b] in Kontakt gebracht wird,
d] danach die Aktivität oder Menge eine Gens oder dessen Genprodukts aus der Zelle aus c] bestimmt wird, wobei dieses Gen oder Genprodukt an Zusammensetzung, Bildung oder Abbau der extrazellulären Matrix beteiligt ist,
e] die Aktivität der Menge aus d] abgeglichen wird mit der Aktivität oder Menge desselben Gens oder Genprodukts aus einer entsprechenden Kontrollzelle, die nicht mit der Verbindung aus b] in Kontakt gebracht wurde,
wobei in d] mindestens ein Gen aus der Gruppe COL1, MMP2, TGF beta 1 oder CTGF gewählt wird.

Die zur Verfügung gestelle Zelle, in der ein Urotensin II Rezeptor gebildet wird, ist bevorzugt die Zelle eines Säugetieres beispielsweise diejenige einer Maus, Ratte, eines Meerschweinchens oder eines Hamsters. Besonders bevorzugt wird eine menschliche Zelle zur Verfügung gestellt. Diese Zelle ist in einer besonders bevorzugten Ausführungsform eine Herzzelle.

Die für das Verfahren zur Verfügung gestellte chemische Verbindung hat bevorzugt ein Molekulargewicht zwischen 100 und 50 000 kDa und besonders bevorzugt zwischen 100 und 5000 kDa.

Solch eine für das Verfahren zur Verfügung gestellte chemische Verbindung ist bevorzugt ein Naturstoff, ein Protein, ein Polynukleotid, eine zuckerhaltige Verbindung oder eine fetthaltige Verbindung. Solch eine Verbindung ist weiterhin bevorzugt Urotensin II.

Das Gen des erfindungsgemäßen Verfahrens oder das entsprechende Genprodukt, für welche Aktivität oder Menge bestimmt werden sollen, ist bevorzugt das Gen oder Genprodukt für Coll (Collagen Typ 1), MMP2 (Metalloproteinase Typ 2), TGFbeta (Transforming Growth Factor beta) oder CTGF (Connective Tissue Growth Factor).

Die Bestimmung der Aktivität oder Menge des Gens oder Genprodukts des erfindungsgemäßen Verfahrens erfolgt bevorzugt mittels quantitativer PCR eines enzymatischen Umsatzes oder mit Hilfe von Antikörpern.

Die Erfindung bezieht sich weiterhin auf die Verwendung einer Verbindung, welche durch ein erfindungsgemäßes Verfahren wie vorstehend beschrieben, identifiziert wurde, zur Herstellung eines Arzneimittels zur Behandlung von Krankheiten des Herz-Kreislauf Systems. Erkrankungen des Herz-Kreislaufsystems sind hierbei beispielsweise der Herzinfarkt, eine koronare Gefäßerkrankung, die krankhafte Veränderung von Blutgefäßen wie z. B. Atherosklerose oder die Angina pectoris. Insbesondere solche Erkrankungen des Herz-Kreislauf Systems sind eingeschlossen, die mit der Bildung von extrazellulärer Matrix in Zusammenhang stehen, wobei diese Bildung krankhaft verändert sein kann.
In einer bevorzugten Ausführungsform zur Verwendung dieser Verbindung hat die Verbindung ein Molekulargewicht zwischen 100 und 50 000 kDa und in einer besonders bevorzugten Ausführungsform ein Molekulargewicht zwischen 100 und 5000 kDa.

Die Modifikation der Aktivität eines Urotensin II Rezeptors bedeutet, daß die von diesem vermittelte Signalweitergabe induziert, gesteigert, abgebrochen oder abgeschwächt werden kann. Die Modifikation der Aktivität eines Urotensin II Rezeptors erfolgt insbesondere durch Agonisten oder Antagonisten von Urotensin II. Die Modifikation des Urotensin II Rezeptors kann aber auch durch sog. allosterische Modulatoren erfolgen. Ein Agonist des Urotensin II ist eine Verbindung, welche die natürlicherweise vorkommende Wirkung des Urotensin II in einer bestimmten Zelle unterstützt aufrechterhält oder steigert, während ein Antagonist diese Wirkung des Urotensin II abschwächt oder aufhebt. Ein allosterischer Modulator des Urotensin II Rezeptors hat kann eine Verbindung sein, die selbst nur geringe Affinität zum Rezeptor besitzt, aber die Wirkung von Agonisten oder Antagonisten potenziert oder hemmt.
Die Aktivität oder Menge eines Gens oder Genprodukts wird beeinflußt, wenn Aktivität oder Menge des betreffenden Gens oder Genprodukts in einer bestimmten Zelle im Vergleich zu einer Kontrolle zunehmen oder abnehmen. Die Kontrolle besteht dann aus einer vergleichbaren Zelle, die den Maßnahmen nicht unterworfen wird, die zur Beeinflussung dieser Aktivität oder Menge führen. Als Maßnahmen seien hierbei insbesondere das Inkontaktbringen einer Verbindung mit der Zelle verstanden. Eine Zelle im oben genannten Sinn kann eine oder mehrere Zellen eines Organs oder Gewebes eines Wirbeltieres bedeuten. Solche Zellen können aus Gehirn, Lunge, Muskel, Fettgewebe, Bindegewebe, dem Herzen oder anderen Organen stammen. Die Zellen können isoliert vorliegen oder noch im Gewebeverband stehen. Solche Zellen umfassen insbesondere auch Zellen aus Zellkulturen. Wirbeltiere sind beispielsweise Ratte, Maus, Hamster, Meerschweinchen und der Mensch.

Urotensin II ist ein Polypeptid. Es besteht beim Menschen aus 11 Aminosäuren. Urotensin II wirkt als sehr effektives gefäßverengendes Hormon. Es bewirkt in Primaten systemische Gefäßverengung, kontraktile Dysfunktion des Myokard und totalen Kreislaufzusammenbruch. Die Wirkung von Urotensin II wird über den GPC-Rezeptor GPR14 vermittelt. Sequenzen für GPR14 sind bekannt. Beispielsweise wird in der internationalen Anmeldung WO 00/75168 die Sequenz des GPR14 der Maus offenbart. Humaner rekombinanter Urotensin II Rezeptor ist kommerziell erhältlich beispielsweise von "Euroscreen".

Die extrazelluläre Matrix (ECM) spielt eine zentrale Rolle bei der Aufrechterhaltung der strukturellen Identität von Geweben. ECM umgibt die Gewebestrukturen oder individuellen Zellen. ECM setzt sich aus Collagenen, nichtcollagenen Glycoproteinen und Proteoglykanen zusammen. Dabei variiert die Zusammensetzung in den unterschiedlichen Geweben. ECM bildet ein hochspezialisiertes, komplexes und dynamisches Netzwerk. Neben seiner stützenden Funktion ist es beteiligt unter anderem bei der Signalübertragung, der Zelldifferenzierung, Adhäsion, Regeneration und Migration. ECM spielt weiterhin eine wichtige Rolle bei der Fibrosierung von Geweben wobei hierunter eine verstärkte Bindegewebseinlagerung verstanden werden soll. Dies erfolgt beispielsweise bei der Vernarbung nach Verletzungen im Herzen nach einem Herzinfarkt, bei der fibrösen Veränderung der Lunge oder fibrösen Veränderung der Leber nach Leberintoxination mit organischen Lösungsmitteln oder übermäßigem und andauerndem Alkoholismus. Hiervon betroffen sind Organe insbesondere auch im Verlauf des fortschreitenden Alterungsprozesses infolge zeitlicher Vorgänge oder durch übermäßige Beanspruchung. Fehlfunktionen in der Bildung der extrazellulären Matrix spielen insbesondere bei der Fibrose eine wichtige Rolle. Fibrose geht einher mit übermäßig starker Vernarbung infolge von Wundheilungsprozessen. Organe wie das Herz, die Nieren, Leber, Lunge, Auge oder die Haut können betroffen sein. Fibrose ist geprägt von vermehrter Bildung von Collagen und Funktionsverlusten der betroffenen Organe.

Am Aufbau des ECM sind Collagene beispielsweise Typ I Collagen oder Typ IV Collagen, Laminine oder andere Proteine beteiligt. Am Abbau der ECM wirken Proteinasen mit, die zur Gruppe der Metalloproteinasen, Serin Proteinasen oder Cystein Proteinasen zu rechnen sind.

Metalloproteinasen weisen charakteristische funktionelle Domänen auf. Signalsequenz, Prodomäne, katalytische Domäne und die C-terminale Hemopexinlike-Domäne sind bei den verschiedenen Mitgliedern der Familie konserviert. MMP2 und MMP9 enthalten zusätzliche Fibronectin-like Domänen. MMP2 ist auch als Gelatinase A bekannt. Substrate der MMP2 sind Collagen I, II, IV, V, VII, X, XI, XIV, Elastin, Fibronectin, Gelatin und Laminin. Der TGFbeta Signalweg und die Aktivierung von CTGF sind Schlüsselprozesse zur Aktivierung von Genen von extrazellulären Matrixproteinen wie dem Collagen Typ I. CTGF wird deshalb auch als fibrogenes Cytokin bezeichnet.

Die Bereitstellung einer Zelle umfaßt deren Herstellung, Anzucht und Weiterverarbeitung. Die Bereitstellung erfolgt beispielsweise durch Präparation geeigneten Zellmaterials aus Organen oder Geweben oder durch die Vermehrung von geeigneten Zelllinien oder Mikroorganismen. Für die Anzucht können verschiedene geeignete Nährmedien verwendet werden. Die Zellen werden bei der für den Organismus optimalen Temperatur gehalten. Gegebenenfalls werden dem jeweils verwendeten Wachstumsmedium Konservierungsmittel, Antibiotika, pH-Indikatoren, Blutserumbestandteile, Blutserum, Hilfsstoffe oder anderes zugegeben. Verfahren zur Herstellung, Anzucht und Weiterverarbeitung sind in Standardwerken beschrieben. (Beispiel: Basic Cell Culture; Ed. J. M. Davis; IRL Press; 1994).

Als Zellen eignen sich bevorzugt solche von Säugetieren. Diese Zellen können aus Primärzellen oder Zelllinien bestehen. Beispiele für solche Zellen sind Primärzellen aus Organen von Säugetieren (z.B. Gehirn, Muskel, Fettgewebe, Bindegewebe, Herz, Lunge, Leber, Niere, Blutgefäße, Hormondrüsen u.a.). Die Zellen können Nervenzellen, Muskelzellen, Fettzellen, Fibroblasten, Leukozyten, Lungenzellen, Leberzellen, Nierenzellen und andere sein. Geeignete Zelllinien sind bevorzugt CHO-, HEK 293, COS-, Maus 3T3-, Hela-Zellen oder andere. Weiterhin bevorzugt können Hefezellen verwendet werden.

Eine chemische Verbindung wird zur Verfügung gestellt, insbesondere durch chemische Synthese oder durch Isolierung chemischer Stoffe aus biologischem Material. Biologisches Material enthält lebende oder nicht lebende Zellen oder Bestandteile von diesen.

Für die chemische Synthese einer Verbindung bzw. die Isolierung eines Stoffes aus biologischen Zellen kann der Fachmann auf Routinemethoden zurückgreifen. Solche Methoden stehen dem Fachmann zur Verfügung in Lehrbüchern wie dem "Organic Synthesis Workbook; 1995; John Wiley & Sons; ISBN 3-527-30187-9", dem "The Organic Chemistry of Drug Synthesis; 1998; John Wiley & Sons; ISBN 0-471-24510-0" oder dem "Bioactive Compounds from Natural Sources; 2001; Taylor & Francis; ISBN 0-7484-0890-8".

Die durch Synthese oder Isolierung gewonnenen Verbindungen können in einem geeigneten Lösungsmittel in Lösung gebracht werden. Geeignete Lösungsmittel können Wasser, Puffersubstanzen (z.B. Tris, Hepes, Mops u.a.) einwertige und/oder zweiwertige Ionen (z.B. K⁺, Na⁺, Mg²⁺, Ca²⁺ u.a.), Säuren (z.B. HCL, H₂SO₄, Ameisensäure, Essigsäure u.a.), Laugen (z.B. NaOH u.a.), Alkohol (z.B. Methanol, Ethanol, Glycerin), Detergenzien (z.B. Na-dodecylsulfat u.a.), organische Lösungsmittel (z.B. Formamid, Azeton, Dimethylsulfoxid u.a.) sowie weitere Bestandteile insbesondere zur Lösungsvermittlung oder Stabilisierung enthalten. Eine chemische Verbindung für das erfindungsgemäße Verfahren sollte sich als Ligand für einen GPCR eignen.

Eine solche Verbindung kann insbesondere aus Geweben oder Organen von Wirbeltieren wie z.B. Endothelzellgewebe, Herzgewebe, Gehirngewebe, Blut, Serum oder Plasma gewonnen werden.

Zum In-Kontakt-Bringen der chemischen Verbindung mit der genannten Zelllinie kann der Fachmann Routinemethoden aus dem Labor verwenden. Das In-Kontakt-Bringen erfolgt beispielsweise in Erlenmayergefäßen, Röhrchen, Eppendorfgefäßen oder auf Mikrotiterplatten. Zum In-Kontakt-Bringen können temperierte Brutschränke verwendet werden, in denen eine konstante Temperatur von beispielsweise 30°C oder 37°C sowie gleichmäßige CO₂-Bedingungen oder Luftfeuchtigkeitsbedingungen einregelbar sind. Das In-Kontakt-Bringen kann insbesondere auch in dafür vorgesehenen Vorrichtungen eines Laborroboters vorgenommen werden. Das In-Kontakt-Bringen ist über unterschiedliche Zeitspannen von wenigen Sekunden, über Minuten bis zu mehreren Stunden möglich. Die jeweils zu wählenden Bedingungen hängen vom Rezeptor, der Zelllinie und der chemischen Verbindung ab.

Die Aktivität oder Menge eines Gens oder dessen Genprodukts kann sich auf die Aktivität des Gens hinsichtlich seines Promoters, der Menge an gebildeter RNA und mRNA sowie der enzymatischen Aktivität oder Menge des von diesem Gen kodierten Proteins beziehen. Die Bestimmung der Menge einer mRNA in einer Zelle kann durch unterschiedliche Methoden erfolgen wie beispielsweise PCR oder Hybridisierungstechniken. Als PCR Techniken stehen dem Fachmann insbesondere die Real-time PCR Systeme zur Verfügung, die von verschiedenen Anbietern kommerziell vertrieben werden. Solche Anbieter sind Applied Biosystems, Bio-Rad, Cepheid, Cortett Research, Roche Molecular Biochemicals oder Stratagene. Real-time PCR Systeme gehören zum standardmäßigen Handwerkszeug des Fachmanns. Als Hybridisierungstechniken zur Bestimmung der RNA Menge eignen sich beispielsweise die Dot-Blot Hybridisierung oder die Northern-Hybridisierung. Beide Techniken machen sich die durch Basenpaarung bewirkte Ausbildung eines doppelsträngigen Nukleinsäuremoleküls aus zwei getrennten Einzelsträngen zunutze, wobei der eine Strang auf einer Trägeroberfläche fixiert wird und der andere mobile Strang eine relativ einfach nachweisbare Markierung radioaktiver oder anderer Art aufweist. Hybridisierungstechniken sind dem Fachmann geläufig. Er findet solche in Standardhandbüchern wie insbesondere in "Current Protocols in Molecular Biology; edited by Fred M. Ausubel, Roger Brent, Robert E. Kingston, David D. Moore, J. G. Seidman, John A. Smith, Keven Struhl; John Wiley & Sons, Inc., New York, 2001.

Die Bestimmung der Aktivität oder Menge eines Proteins kann über dessen enzymatische Aktivität oder mit Hilfe von spezifischen Antikörpern erfolgen. Bei der enzymatischen Aktivität wird der Umsatz des jeweiligen Enzyms bezüglich seines Substrats bestimmt. Dazu wird ein Assay aufgebaut, der hinsichtlich pH, Cofaktoren, lonenmilieu, Temperatur und der Substratkonzentration dem Enzym optimale Aktivitätsbedingungen gewährleistet und eine Messung innerhalb des proportionalen Umsatzbereichs ermöglicht. Die Bestimmung des Substratumsatzes kann beispielsweise nach dem Einbau radioaktiv markierter Vorstufen in Makromoleküle oder die Zerlegung radioaktiv markierter Makromoleküle in Abbauprodukte, deren nachfolgende Trennung und Bestimmung der jeweiligen radioaktiven Mengen erfolgen. Beim Einsatz von Antikörpern zur Mengenerfassung eines Proteins bedient sich der Fachmann Techniken wie der Southern-Hybridisierung, RIA (Radio-Immun-Assay), ELISA (Enzym linked immunosorbent assay) und andere.
Auch zu diesen Nachweismethoden findet der Fachmann in vorstehend genannten "Current Protocols in Molecular Biology" detaillierte praktische Anleitungen zur Durchführung.

Die Verbindung liegt im Arzneimittel vorzugsweise mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05 % bis 95 Gew.-% des Wirkstoffes enthalten kann.
Die erfindungsgemäßen pharmazeutischen Zusammensetzungen können nach einer der bekannten pharmazeutischen Methoden hergestellt werden, die im wesentlichen darin bestehen, die Bestandteile mit pharmakologisch verträglichen Träger- und/oder Hilfsstoffen zu mischen. Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichungen geeignet sind.

Die Menge einer Verbindung, die erforderlich ist, um einen gewünschten biologischen Effekt zu erzielen, ist abhängig von einer Reihe von Faktoren wie z.B. der gewählten spezifischen Verbindung, der beabsichtigten Verwendung, der Art der Verabreichung oder dem klinischen Zustand des Patienten. Im folgenden genannte Mengenangaben beziehen sich auf einen Liganden.

Im allgemeinen liegt die Tagesdosis im Bereich von 0,3 mg bis 100 mg (typischerweise von 3 mg bis 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichte Einzeldosisformulierungen, wie zum Beispiel Tabletten oder Kapseln, beispielsweise von 1,0 bis 1000 mg, typischerweise von 10 bis 600 mg enthalten.

### Abkürzungen

| | |
|---|---|
| All | Angiotensin II |
| Coll | Collagenase I |
| Con | Kontrolle |
| MMP2 | Matrix Metalloproteinase II |
| UII | Urotensin II. |

### Beispiele

### 1. Isolierung und Kultivierung von Fibroblasten aus menschlichen Herzen

Die Isolierung von Fibroblasten aus menschlichen Herzen erfolgte wie beschrieben in Cell and Tissue Research 286, 145-153 (1996). Die resultierende Zellkultur bestand zu 96-98 % aus Fibroblasten.
Zur Durchführung wurde Herzgewebe in kleine Stücke geschnitten und dreimal in KHB (Krebs Henseleit Puffer) enthaltend 2 mg/ml Kollagenase und 2 mg/ml Dispase für 20 Minuten bei 37°C inkubiert. Die Homogenate wurden zusammen gegeben und anschließend zentrifugiert. Die Sedimente wurden in 10 ml KHB aufgenommen. Die Zellen wurden in jeweils 2 bis 3 ml Portionen in nicht beschichtete Kunststoffbehälter gegeben. Das Volumen wurde mit DMEM (Dulbecco's Modification of Eagle's Medium) enthaltend 4,5 mg/l Glukose und 10 % FCS (fötales Kälberserum) zu 10 ml ergänzt. Nach Inkubation für 1 Stunde bei 37 °C wurden die Zellen zweimal mit PBS (Phosphat gepufferte Saline) gewaschen und anschließend für ca. 4-8 Wochen bei 37°C und 5 % CO₂ kultiviert, bis 90 % Konfluenz erreicht war. Daraufhin folgten zwei weitere Passagen von jeweils 4 Wochen Dauer. Die Zellen der hier vorliegende Versuche wurden nach der zweiten Passage bei 70 % bis 90 % Konfluenz verwendet (ca. 3 Monate nach Isolierung).

### 2. Stimulierung von Fibroblasten aus menschlichen Herzen durch Angiotensin II und Urotension II

Die Reagenzien für die Durchführung der Zellkulturtechniken wurden von Gibco BRL Life Technologies bezogen. Die Zellen wurden zuerst in serumfreiem DMEM enthaltend 4000 mg Glucose/I, 2mM L-Glutamin, 100 µg/0,1 mg/ml Penicillin/Streptomycin und 0,1 % BSA (Rinderserumalbumin) bei 37 °C gehalten bevor Angiotensin II oder Urotensin II bis jeweils 0,1 µM Endkonzentration zugegeben wurde. Anschließend wurde nach 6 Stunden RNA aus den Zellen präpariert.

### 3. Durchführung der Quantifizierung spezifischer RNAs durch Real time PCR

Die RNA wurde mittels Standardverfahren unter Zuhilfenahme von Guanidiniumchlorid isoliert.
Von jeder Probe wurden 500 ng der RNA mit reverser Transkriptase (10units/ng RNA) in DNA umgeschrieben. Zum Abbau der DNA wurde das gesamte Volumen mit ca. 8-10 µg RNA mit DNase I inkubiert.
Die Quantifizierung der RNAs erfolgte mit dem Light-Cycler und einem Fast Start Sybr-Green RT-PCR Kit von Roche Molecular Biochemicals. Für denselben Zweck eignen sich auch entsprechende Kits anderer Firmen wie Applied Biosystems, Bio-Rad oder Stratagene. Als Primer wurden für Col I sie Primer entsprechend SEQ ID No. 1 und 2, für MMP2 die Primer entsprechend SEQ ID No. 3 und 4, für GAPDH als Kontrolle die Primer entsprechend SEQ ID No. 5 und 6, für TGFbeta die Primer entsprechend SEQ ID Nr. 7 und 8 und für CTGF die Primer entsprechend SEQ ID Nr. 9 und 10 verwendet. Die Reaktion wurde zuerst für 10 Min. bei 95°C konstant gehalten. Dann folgten 40 Zyklen, wobei sich jeder Zyklus aus einer Inkubation für 10 Sek. bei 95°C, 5 Sek. bei 60°C und 12 Sek. bei 72°C zusammensetzte. Die Spezifität der Reaktion wurde durch eine Schmelzkurve bestätigt. Der Abgleich der Mengen erfolgte mit GAPDH mRNA als Kontrolle.

### 4. Induktion von Genen, die am Aufbau der Basalmembran beteiligt sind, in Fibroblasten aus dem Herz

Fibroblasten wurden aus drei menschlichen Herzen isoliert und in der ersten Passage verwendet. Die Zellen wurden bis ca. 80 % Konfluenz kultiviert, für 24 weitere Stunden ohne Serum gehalten und mit 1 µM Urotensin II, 1 µM Angiotensin II oder Puffersubstanzen als Kontrollen über 6 Stunden jeweils in doppelten Ansätzen stimuliert. Die mRNAs (Collagen1, Matrix Metalloproteinase 2, TGFbeta1, CTGF) wurden quantifiziert mittels "Real time PCR" unter Verwendung eines Light Cyclers^{™}. Als Standardreferenz wurde die mRNA von GAPDH (Glyceraldehyde-3-phosphate Dehydrogenase) verwendet. Real time PCR (Polymerase chain reaction in Echtzeit) ermöglicht die quantitative Erfassung spezifischer Nukleinsäuren. Die Technik basiert auf der kombinierten Anwendung von fluoreszierenden Farbstoffen mit der Polymerase chain reaction von revers transkribierter RNA. Es gibt mittlerweile unterschiedliche Anbieter von Assay Kits. Das System ist beispielsweise kommerziell erhältlich von Applied Biosystems, Bio-Rad, Cepheid, Carbett Research, Roche Molecular Biochemicals oder Stratagene.

Die Stimulierung durch Urotensin II führte zu signifikantem Anstieg der mRNAs von Collagen I, MMP2, TGFbeta1 und CTGF

| | Col 1 | MMP 2 | TGFbeta1 | CTGF |
|---|---|---|---|---|
| Con | 0,56+/-0,1 | 0,43+/-0,2 | 0,60+/-0,09 | 0,45+/-0,27 |
| UII | 0,92+/-0,3* | 0,65+/-0,3* | 0,81+/-0,1* | 0,88*/-0,2* |
| All | 0,56+/-0,27 | 0,22+/-0,1 | 0,71+/-0,07 | 0,34+/-0,9 |

| | | | | |
|---|---|---|---|---|
| *: p< 0.05 vs control; | | | | |

Die Bildung dieser RNAs zeigt die enorme Bedeutung von Urotensin II und dessen Rezeptor für Matrixsynthese und Gewebeumbau an Gefäßen und dem Herz. Entsprechende Agonisten und Antagonisten haben deshalb großen pharmakologischen Wert bezüglich Umbildungen am Herz insbesondere nach einem Herzschlag in den betroffenen Bezirken zur Vorbeugung, Verhinderung oder Umkehrung der Vernarbung am Herzgewebe, insbesondere aber auch bei Bluthochdruck oder obstruktiven Lungenkrankheiten. Angiotensin II ist das Haupteffektormolekül des Renin-Angiotensin Systems. Das Peptidhormon bewirkt unterschiedliche physiologische Effekte. So ist Angiotensin II beteiligt an der Änderung der peripheren Resistenz. Es hat Einfluß auf die Nierenfunktion und steuert unter anderem die Natrium-Rücksorption. Darüber hinaus wirkt es als Wachstumsfaktor.

### 5. Induktion der Collagensynthese durch Urotensin in humanen cardialen Fibroblasten

Humane cardiale Fibroblasten wurden isoliert aus sieben explantierten humanen Herzen im Endstadium der Herzinsuffizienz aufgrund dilatativer Kardiomyopathie. Ebenso wurden vier Zellpräparationen aus gesunden Donorherzen präpariert. Die Fibroblasten wurden in sterilen 10 cm Petrischalen isoliert und kultiviert wie vorstehend beschrieben. Die Zellen erreichten nach etwa 12 wöchiger Kultivierung die zweite Passage mit ca. 80 % Konfluenz. In diesem Stadium wurde für 48 Stunden das Serum entzogen und anschließend für verschiedene Zeitintervalle (von 6 bis 24 Stunden) mit UII, Ang II, TNFalpha, TGFβ, UII + TNFa, (jeweils 10⁻⁷ mol/I) bzw. Puffer (Kontrolle) stimuliert. Es wurden Doppel- oder Dreifachbestimmungen durchgeführt. Nach der Stimulation wurden die Zellen gewaschen, mittels 1 ml PBS aus den Petrischalen entfernt und in Eppendorfgefäßen abzentrifugiert (100 rpm, 5 min, 4°C). Der Überstand wurde verworfen. Zu etwa 50 ul Zellen wurden 100 ul Lysepuffer gegeben (Nonident P40, 0.5%, SDS 0.5%) und für weitere 20 Minuten auf Eis inkubiert. Das Lysat wurde erneut zentrifugiert (20 Minuten, 1400 rpm, 4°C) und der Überstand für die weiteren Experimente verwendet. Die Protein Bestimmung wurde durchgeführt mit dem BCA Assay (Bicinchoninic Acid). Die Homogenate wurden verdünnt auf eine Proteinkonzentration von 2 ug/ul.
Procollagen Typ I C-peptide der Fibroblasten aus allen Herzen wurde bestimmt mittels eines kommerziell erhältlichen Festphasen-ELISA Assay auf einer 96well Mikrotiterplatte (TaKaRa Biomedicals, Shuzo Co, Ltd.). Der Assay wurde nach den Angaben des Herstellers durchgeführt. Dabei zeigte sich keine Kreuzreaktivität mit humanem Fibronectin, Vitronectin, Laminin, Collagen Typ I oder Collagen Typ III. Die Bestimmung der Werte für das Procollagen Typ I C-peptide wurden bestimmt mit Hilfe einer Standardkurve von 10-640 ng/ml. Die Standards werden vom Hersteller zur Verfügung gestellt. Die Absorptionswerte der Stimulationen aus den Kontrollen (Puffer) wurden als 100% gesetzt. Die Absorptionswerte der stimulierten Zellen wurden zu den Kontrollwerten desselben Herzens in Bezug gesetzt. Es wurden jeweils die Mittelwerte aus zwei- oder Dreifachbestimmungen für die Berechnungen herangezogen. Zur statistischen Auswertung des Vergleichs der Stimulationsantworten in Kontrollzellen und Herzinsuffizienzzellen wurde ein zweiseitiger, nicht gepaarter T-Test verwendet.

Procollagen I Protein ist erhöht in den explantierten Herzfibroblasten der Herzen mit Herzversagen nach Stimulation mit UII (173 +/- 42 % der Kontrolle), Ang II (205 +/- 67 %), TGF β (202 +/- 52 %) und in geringerem Ausmaß mit TNFalpha (139 +/- 26 %) (Tabelle 1). Die Zunahme erfolgte zeitabhängig, wurde aber bereits nach 6 Stunden beobachtet. Im Gegensatz dazu war die Kollagenproteinmenge nicht verändert in den Fibroblasten der Donorherzen, die mit denselben Peptiden stimuliert wurden: UII (98 % der KOntrolle), Ang II (88 %), TGFβ (97 %) and TNFalpha (86 %). Die Unterschiede zwischen Zellen der Kontrollherzen und Zellen der Herzen mit Herzinsuffizienz waren statistisch signifikant nach Stimulation mit UII und TNFalpha. Die gleichen Tendenzen wurden auch mit Angll und TGFbeta beobachtet.

**Tabelle 1**

| Kollagensynthese in Fibroblasten von Herzen im Endstadium der Herzinsuffizienz (EFH) | | | | | | | |
|---|---|---|---|---|---|---|---|
| | | | | | | | |

| Herz | Zeit | Kontr | Ang II | TGFβ | UII | TNFa | UII+TNF |
|---|---|---|---|---|---|---|---|
| (Nr.) | (Std.) | (Absorption, in % der Kontrollen) | | | | | |
| 1 | 24 h | 100 | 142 | 166 | 198 | 166 | |
| 4 | 24 h | 100 | 471 | 377 | 458 | 262 | |
| 10 | 5 h | 100 | 94 | 142 | 102 | 67 | |
| 11 | 5h | 100 | 113 | 123 | 243 | 91 | |
| 12 | 12 h | 100 | - | - | 117 | 107 | 128 |
| 13 | 12 h | 100 | - | - | 165 | 126 | 139 |
| 14 | 12 h | 100 | - | - | 226 | 190 | 218 |
| MW | | 100 | 205 | 202 | 173 | 139 | 162 |
| SEM | | | 67 | 52 | 42 | 26 | 18 |
| | | | | | | | |

| Collagen Synthese in Kontrollherzen (Kontr) | | | | | | | |
|---|---|---|---|---|---|---|---|
| | | | | | | | |

| Herz | Zeit | Kontr | Ang II | TGFβ | UII | TNFa | |
|---|---|---|---|---|---|---|---|
| (Nr.) | (Std.) | (Absorption, in % der Kontrollen) | | | | | |
| 3 | 6h | 100 | 84 | 84 | 103 | 81 | |
| 3 | 12h | 100 | 92 | 123 | 80 | 92 | |
| 5 | 6h | 100 | 88 | 71 | 106 | 77 | |
| 6 | 6h | 100 | - | 110 | 104 | 92 | |
| MW | | 100 | 88 | 97 | 98 | 86 | |
| SEM | | | 2 | 12 | 6 | 4 | |
| | | | | | | | |
| p (EFH gegen Kontrollen) 0.3 0.16 0.05 0.02 | | | | | | | |
| | | | | | | | |
| (Nr.) : interne Code Nummer für die Herzen. | | | | | | | |

## Patentansprüche

1. Verfahren in vitro zur Identifizierung einer Verbindung, welche die Aktivität des Urotensin II Rezeptors so modifiziert, dass Aktivität oder Menge eines Gens oder Genprodukts, welches an Zusammensetzung, Bildung oder Abbau der extrazellulären Matrix beteiligt ist, beeinflusst werden,
in dem
a] eine Zelle zur Verfügung gestellt wird, in der ein Urotensin II Rezeptor gebildet wird,
b] eine chemische Verbindung zur Verfügung gestellt wird,
c] die Zelle aus a] mit der chemischen Verbindung aus b] in Kontakt gebracht wird,
d] danach die Aktivität oder Menge eines Gens oder dessen Genprodukts aus der Zelle aus c] bestimmt wird, wobei dieses Genprodukt an Zusammensetzung, Bildung oder Abbau der extrazellulären Matrix beteiligt ist,
e] die Aktivität oder Menge aus d] abgeglichen wird mit der Aktivität oder Menge desselben Gens oder Genprodukts aus einer entsprechenden Kontrollzelle, die nicht mit der Verbindung aus b] in Kontakt gebracht wurde,
wobei in d) mindestens ein Gen aus der Gruppe Col 1, MMP2, TGFbeta1 oder CTGF gewählt wird.

2. Verfahren nach Anspruch 1, wobei die Zelle, die zur Verfügung gestellt wird, eine Säugetierzelle ist.

3. Verfahren nach einem oder mehreren der Ansprüche 1 bis 2, wobei die Zelle, die zur Verfügung gestellt wird, eine humane Zelle ist.

4. Verfahren nach einem oder mehreren der Ansprüche 1 bis 3, wobei die Zelle, die zur Verfügung gestellt wird, eine Herzzelle oder ein Fibroblast ist.

5. Verfahren nach einem oder mehreren der Ansprüche 1 bis 4, wobei die Verbindung, die zur Verfügung gestellt wird, ein Molekulargewicht zwischen 100 und 50 000 kDa hat.

6. Verfahren nach Anspruch 5, wobei das Molekulargewicht zwischen 100 und 5000 kDa liegt.

7. Verfahren nach Anspruch 5 oder 6, wobei die Verbindung ein Naturstoff, ein Protein, ein Polynukleotid, eine zuckerhaltige Verbindung oder eine fetthaltige
Verbindung ist.

8. Verfahren nach Anspruch 7, wobei die Verbindung Urotensin II ist.

9. Verfahren nach einem oder mehreren der Ansprüche 1 bis 8, wobei die Bestimmung gemäß d] oder e] mittels quantitativer PCR erfolgt.

10. Verfahren nach einem oder mehreren der Ansprüche 1 bis 8, wobei die Bestimmung gemäß d] oder e] mittels eines enzymatischen Umsatzes erfolgt.

11. Verfahren nach einem oder mehreren der Ansprüche 1 bis 8, wobei die Bestimmung gemäß d] oder e] mittels eines Antikörpers erfolgt.

## Claims

1. A method in vitro for identifying a compound which modifies the activity of the urotensin II receptor such that the activity or quantity of a gene or gene product which is involved in the structure, formation or breakdown of the extracellular matrix is influenced,
in which
a] a cell in which a urotensin II receptor is formed is made available,
b] a chemical compound is made available,
c] the cell from a] is brought into contact with the chemical compound from b],
d] after that, the activity or quantity of a gene, or its gene product, from the cell from c] is determined, with this gene product being involved in the structure, formation or breakdown of the extracellular matrix,
e] the activity or quantity from d] is compared with the activity or quantity of the same gene or gene product from a corresponding control cell which has not been brought into contact with the compound from b],
wherein at least one gene from the group Col 1, MMP2, TGFbeta1 and CTGF is selected in d].

2. The method as claimed in claim 1, wherein the cell which is made available is a mammalian cell.

3. The method as claimed in one or both of claims 1 and 2, wherein the cell which is made available is a human cell.

4. The method as claimed in one or more of claims 1 to 3, wherein the cell which is made available is a heart cell or a fibroblast.

5. The method as claimed in one or more of claims 1 to 4, wherein the compound which is made available has a molecular weight of between 100 and 50 000 kDa.

6. The method as claimed in claim 5, wherein the molecular weight is between 100 and 5000 kDa.

7. The method as claimed in claim 5 or 6, wherein the compound is a natural product, a protein, a polynucleotide, a sugar-containing compound or a fat-containing compound.

8. The method as claimed in claim 7, wherein the compound is urotensin II.

9. The method as claimed in one or more of claims 1 to 8, wherein the determination in accordance with d] or e] is effected by means of quantitative PCR.

10. The method as claimed in one or more of claims 1 to 8, wherein the determination in accordance with d] or e] is effected by means of an enzymic turnover.

11. The method as claimed in one or more of claims 1 to 8, wherein the determination in accordance with d] or e] is effected by means of an antibody.

## Revendications

1. Procédé pour l'identification in vitro d'un composé qui modifie l'activité du récepteur de l'urotensine II de manière telle que l'activité ou la quantité d'un gène ou d'un produit génique, qui participe à la composition, à la formation ou à la dégradation de la matrice extracellulaire, sont influencées, dans lequel
a] on met à disposition une cellule dans laquelle est formé un récepteur d'urotensine II,
b] on met à disposition un composé chimique,
c] on met en contact la cellule de a] avec le composé chimique de b],
d] on détermine ensuite l'activité ou la quantité d'un gène ou de son produit génique de la cellule de c], ce produit génique participant à la composition, à la formation ou à la dégradation de la matrice extracellulaire,
e] on compare l'activité ou la quantité de d] avec l'activité ou la quantité du même gène ou produit génique d'une cellule de contrôle correspondante, qui n'a pas été mise en contact avec le composé de b],
en choisissant dans d] au moins un gène du groupe Col 1, MMP2, TGFbeta1 ou CTGF.

2. Procédé selon la revendication 1, où la cellule qui est mise à disposition est une cellule d'un mammifère.

3. Procédé selon l'une ou plusieurs des revendications 1 à 2, où la cellule qui est mise à disposition est une cellule humaine.

4. Procédé selon l'une ou plusieurs des revendications 1 à 3, où la cellule qui est mise à disposition est une cellule du coeur ou un fibroblaste.

5. Procédé selon l'une ou plusieurs des revendications 1 à 4, où le composé qui est mis à disposition présente un poids moléculaire entre 100 et 50 000 kDa.

6. Procédé selon la revendication 5, où le poids moléculaire est situé entre 100 et 5000 kDa.

7. Procédé selon la revendication 5 ou 6, où le composé est une substance naturelle, une protéine, un polynucléotide, un composé contenant du sucre ou un composé contenant de la graisse.

8. Procédé selon la revendication 7, où le composé est l'urotensine II.

9. Procédé selon l'une ou plusieurs des revendications 1 à 8, où la détermination selon d] ou e] est réalisée au moyen d'une PCR quantitative.

10. Procédé selon l'une ou plusieurs des revendications 1 à 8, où la détermination selon d] ou e] est réalisée au moyen d'une conversion enzymatique.

11. Procédé selon l'une ou plusieurs des revendications 1 à 8, où la détermination selon d] ou e] est réalisée au moyen d'un anticorps.


### SEQUENZPROTOKOLL

<110> Aventis Pharma Deutschland GmbH
<120> Verfahren zur Identifizierung von Verbindungen zur Therapie von Alterungsprozessen des Herz-Kreislauf Systems
<130> AVE D-2001/A032
<140>
   <141>
<160> 10
<170> PatentIn Ver. 2.1
<210> 1
   <211> 20
   <212> DNA
   <213> Homo sapiens
<400> 1
   cgttggacct cctggtaatc 20
<210> 2
   <211> 20
   <212> DNA
   <213> Homo sapiens
<400> 2
   ccttgttacc gctctctcct 20
<210> 3
   <211> 20
   <212> DNA
   <213> Homo sapiens
<400> 3
   acctggatgc cgtcgtggac 20
<210> 4
   <211> 20
   <212> DNA
   <213> Homo sapiens
<400> 4
   tgtggcagca ccagggcagc 20
<210> 5
   <211> 18
   <212> DNA
   <213> Homo sapiens
<400> 5
   gcaggaggca ttgctgat 18
<210> 6
   <211> 20
   <212 DNA
   <213> Homo sapiens
<400> 6
   caccatcttc caggagcgag 20
<210> 7
   <211> 20
   <212> DNA
   <213> Homo sapiens
<400> 7
   gttgtaatgg caggcacagg 20
<210> 8
   <211> 23
   <212> DNA
   <213> Homo sapiens
<400> 8
   cacactgcaa gtggacatca acg 23
<210> 9
   <211> 24
   <212> DNA
   <213> Homo sapiens
<400> 9
   tctccgtgga gctgaagcaa tagt 24
<210> 10
   <211> 20
   <212> DNA
   <213> Homo sapiens
<400> 10
   ggcttaccga ctggaagaca 20